# EUROPÄISCHE PATENTSCHRIFT

(11) **EP 0 367 252 B1**
(45) Veröffentlichungstag und Bekanntmachung des Hinweises auf die Patenterteilung: **21.07.1993**
(21) Anmeldenummer: 89120236.8
(22) Anmeldetag: 01.11.1989
(51) Int. Cl.: A61M 1/16

(54) **Verfahren und Vorrichtung zur Ultrafiltration bei der Hämodialyse**
Method and apparatus for ultrafiltration during hemodialysis
Procédé et dispositif d'ultrafiltration dans l'hémodialyse

(30) Priorität: 04.11.1988 DE 3837498
(43) Veröffentlichungstag der Anmeldung: 09.05.1990
(73) Patentinhaber: Fresenius AG, 61350 Bad Homburg (DE)
(72) Erfinder: Polaschegg, Hans-Dietrich, Dr., D-6370 Oberursel 4 (DE)
(74) Vertreter: Fuchs Mehler Weiss

(56) Entgegenhaltungen:
- FR-A- 2 434 624
- US-A- 4 676 905

## Beschreibung

Die Erfindung betrifft ein Verfahren zum Betreiben einer Hämodiafiltrationsvorrichtung, bei dem die Dialysierflüssigkeit durch eine an den Dialysator angeschlossene mit Absperrventilen versehene und Bilanzkammern aufweisende Bilanziereinrichtung geleitet wird und das Ultrafiltrat in einer genau vorherbestimmten Menge entzogen wird. Die Erfindung betrifft eine Vorrichtung zur Durchführung des Verfahrens.

Aus der DE-PS 28 38 414 (FR-A-24 34 624) ist eine Vorrichtung bekannt, die eine Bilanziereinrichtung aufweist, die aus zwei durch ein verschiebbares Element getrennten Kammern besteht, die je eine Zuführleitung für frische und eine mit einem Aus lauf verbundene Abführleitung für gebrauchte Dialysierflüssigkeit aufweist. In den Zuführ- und Abführleitungen sind Absperrventile angeordnet, die von einer Steuereinheit angesteuert und geschaltet werden. Zum Fördern der verbrauchten Dialysierflüssigkeit ist im Dialysierflüssigkeitsweg zwischen Dialysator und Bilanzeinrichtung eine Pumpe angeordnet. Weiterhin sieht diese bekannte Vorrichtung in der Zuführleitung zum Dialysator ein Dialysatorventil und in der Abführleitung des Dialysators einen Luftabscheider vor.

Diese Vorrichtung wird derart betrieben, daß frische Dialysierflüssigkeit von einer Dialysierflüssigkeitsquelle den beiden Bilanzkammern abwechselnd durch entsprechende Schaltung der Absperrventile in den Zuführleitungen zugeführt wird. Gleichzeitig wird frische Dialysierflüssigkeit aus einem bereits gefüllten Raum der anderen Bilanzkammer dem Dialysator zugeführt, wo durch Diffusion dem ebenfalls durch den Dialysator fließenden Blut die gewünschten Giftstoffe entzogen werden. Die durch diese Diffusion verbrauchte Dialysierflüssigkeit wird anschließend in den zweiten Raum derselben Bilanzkammer gepumpt, von wo aus die verbrauchte Dialysierflüssigkeit dann in den Auslauf geleitet wird.

Der zwischen der Bilanzeinrichtung und dem Dialysator eingeschlossene Teil des Flüssigkeitkreislaufes verhält sich wie ein geschlossenes, volumenkonstantes System. Um aus diesem System Flüssigkeit abzuführen, ist eine Entnahmevorrichtung vorgesehen, die an einen Ablauf angeschlossen ist.

Die mit Hilfe der Entnahmevorrichtung aus dem System abgeleitete Flüssigkeitsmenge muß aufgrund der erwähnten Eigenschaften der Bilanziervorrichtung durch eine gleich große Flüssigkeitsmenge ersetzt werden, die von der Blutseite zur Dialysierflüssigkeitsseite der Dialysatormembran übergeht. Die mittels der Entnahmevorrichtung abgeleitete Flüssigkeitsmenge stimmt also mit der durch die Membran des Dialysators tretenden Flüssigkeitsmenge, dem Ultrafiltrat, überein. Die Entnahmevorrichtung, die im wesentlichen eine Ultrafiltratpumpe aufweist, ist so ausgestaltet, daß eine Steuerung der Ultrafiltration möglich ist.

An die Genauigkeit der Bilanziervorrichtung und der Entnahmevorrichtung sind hohe Anforderungen zu stellen. Bei einer Hämodialysebehandlung werden typischerweise etwa 200 l Dialysierlösung durch den Dialysator geleitet. Die Ultrafiltratmenge beträgt typischerweise etwa 2 - 3 l und sollte bis auf eine Abweichung in der Größenordnung 0,1 bis 0,2 l genau bestimmbar sein. Der in der Bilanziereinrichtung verursachte Bilanzierungsfehler darf demnach die Größenordnung von 1 Promille möglichst nicht überschreiten. Um auch eine genau vorherbestimmbare Menge Ultrafiltrat dem Dialysierflüssigkeitsweg mit der erforderlichen Genauigkeit zu entziehen, ist die Entnahmevorrichtung mit einer volumetrischen Membranpumpe ausgerüstet, wobei jeder einzelne Pumpenhub einer Einheitsmenge Ultrafiltrat entspricht. Die Entnahme erfolgt über eine Leitung aus dem unteren Teil eines im Dialysatorkreislauf angeordneten Luftabscheiders, um sicherzustellen, daß nur blasenfreie Flüssigkeit abgeführt wird. Der Ausgang der Ultrafiltrationspumpe bzw. der Entnahmevorrichtung ist über ein Umschaltventil mit der Abflußleitung verbunden.

Obwohl diese bekannte Vorrichtung genau und zuverlässig funktioniert, besteht dennoch das Bedürfnis, Geräte dieser Art technisch zu vereinfachen, d.h. insbesondere die Zahl der aktiven Komponenten zu vermindern und Kalibriervorgänge und sicherheitstechnische Kontrollen, die von Zeit zu Zeit durchgeführt werden müssen, einzusparen. Diese Kalibriervorgänge und Kontrollen betreffen auch die Entnahmevorrichtung für das Ultrafiltrat.

Aufgabe der Erfindung ist daher ein Verfahren, mit dem eine vorher bestimmbare Ultrafiltration auf einfachere Weise ohne zusätzliche Ultrafiltratpumpe durchgeführt werden kann. Es ist auch Aufgabe der Erfindung, eine Vorrichtung zur Durchführung dieses Verfahrens bereitzustellen.

Diese Aufgabe wird durch den kennzeichnenden Teil von Anspruch 1 sowie den kennzeichnenden Teil des Anspruchs 8 gelöst.

Während bisher das Ultrafiltrat über eine separate Entnahmevorrichtung im Dialysierflüssigkeitsweg zwischen Dialysatorausgang und Bilanziereinrichtung entnommen wurde, wird erfindungsgemäß das Ultrafiltrat vor dem Abführen aus dem Dialysatorkreislauf zunächst über die Bilanziereinrichtung geführt. Auf diese Weise kann die gesamte Entnahmeeinrichtung für das Ultrafiltrat eingespart werden. Von der Bilanziereinrichtung wird dann das Ultrafiltrat ebenso wie die verbrauchte Dialysierflüssigkeit dem Auslauf zugeführt. Neben der Einsparung der Ultrafiltratpumpe bietet dieses Verfahren weiterhin den Vorteil, daß das Füllvolumen der Bilanzkammern zur Bestimmung der Ultrafiltratmenge eingesetzt werden kann. Da das Füllvolumen einer Bilanzkammer üblicherweise 30 ml umfaßt, kann durch mehrmaliges periodisches Füllen der Bilanzkammer mit Ultrafiltrat die gewünschte Ultrafiltratmenge, die 2 - 3 l beträgt, auf exakte Weise bestimmt und dem Dialysierflüssigkeitsweg entzogen werden.

Gemäß einer ersten Ausführungsform werden zur Durchführung der Ultrafiltration die Absperrventile in den Zuführ- und Abführleitungen der Bilanzkammern zunächst so geschaltet, daß eine Bilanzkammer vollständig mit frischer Dialysierflüssigkeit und die andere Bilanzkammer vollständig mit verbrauchter Dialysierflüssigkeit gefüllt ist. Dann wird der Dialysierflüssigkeitsweg zwischen der Bilanziereinrichtung und dem Dialysator unterbrochen. Dies geschieht zweckmäßigerweise mittels des sogenannten Dialysatorventils. Nachdem dieses Dialysatorventil geschlossen worden ist, wird mittels der Dialysierflüssigkeitspumpe weiterhin verbrauchte Dialysierflüssigkeit aus dem Dialysator gepumpt, wobei sich nach relativ kurzer Zeit aufgrund des unterbrochenen Zuflusses von frischer Dialysierflüssigkeit in der Dialysatorkammer ein Unterdruck einstellt. Dadurch wird dem Blut Flüssigkeit, das sog. Ultrafiltrat, entzogen, das nunmehr von der Dialysierflüssigkeitspumpe in den Raum der Bilanzkammer gepumpt wird, deren zweiter Raum mit frischer Dialysierflüssigkeit gefüllt ist. Da durch die Unterbrechung des Dialysierflüssigkeitsweges die verdrängte frische Dialysierflüssigkeit nicht dem Dialysator zugeführt werden kann, wird durch geeignete Schaltung der Zuführ- und Abführventile der Bilanzkammern die frische Dialysierflüssigkeit in den leeren Raum der anderen Bilanzkammer umgelenkt, wodurch gleichzeitig die in dieser Kammer befindliche verbrauchte Dialysierflüssigkeit zum Auslauf befördert wird. Nachdem die erste Bilanzkammer vollständig mit Ultrafiltrat gefüllt worden ist, ist der erste Durchgang der Ultrafiltration beendet und die Zuführ- und Abführventile der Bilanzkammern werden wieder auf Hämodialysebetrieb geschaltet. Durch Unterbrechung der Hämodialyse kann auf diese Weise jeweils ein Ultrafiltratzyklus eingeschoben werden.

Dieses Verfahren, das den ungleichmäßigen Entzug von Ultrafiltrat bewirkt, kann gemäß einer weiteren Ausführungsform dahingehend verbessert werden, daß das Ultrafiltrat kontinuierlich entzogen wird. Hierzu wird dem Dialysierflüssigkeitsweg vor der Durchführung von Ultrafiltration und Hämodialyse eine solche Menge Luft zugeführt, die dem Füllvolumen der Bilanziereinrichtung oder einem Mehrfachen entspricht. Die gewünschte Luftmenge wird in den üblicherweise zwischen dem Dialysator und der Bilanziereinrichtung im Dialysierflüssigkeitsweg angeordneten Luftabscheider eingeleitet. Hierbei wird ebenfalls der Dialysierflüssigkeitsweg so lange unterbrochen, bis die gewünschte Luftmenge sich im Luftabscheider befindet.

Die Luftzufuhr kann auf zweierlei Weise durchgeführt werden. Gemäß einer bevorzugten Ausführungsform ist an den Luftabscheider eine Luftpumpe angeschlossen, die nach Unterbrechung des Dialysierflüssigkeitswegs zwischen der Bilanzierreinrichtung und dem Dialysator durch Schließen des Dialysatorventils die gewünschte Luftmenge in den Luftabscheider pumpt. Hierbei bleibt die Dialysierflüssigkeitspumpe ebenfalls in Betrieb. Durch geeignete Abstimmung der Förderraten der beiden Pumpen wird ein Druckanstieg im Dialysierflüssigkeitsweg vermieden. Im Luftabscheider sind zwei im Abstand angeordnete Sensoren oder ein analoger Füllstandsmesser derart angeordnet, daß die beiden Sensoren bzw. die Minimal- und Maximalstellung des Füllstandssensors etwa das Füllvolumen einer Bilanzierkammer einschließen. Während dieses Vorgangs wird also keine Flüssigkeit aus dem Blut entzogen, sondern das geschlossene System kontrolliert teilweise mit Luft gefüllt. Dieser Vorgang erfordert nur wenige Sekunden Zeit. Anschließend werden die Ventile wieder in den Dialysemodus geschaltet und die Luftpumpe beginnt nun in umgekehrter Richtung zu arbeiten, um die zuvor in den Dialysierflüssigkeitsweg gepumpte Luft wieder aus dem Luftabscheider zu entfernen, wodurch eine Ultrafiltration von Flüssigkeit aus dem Blut hervorgerufen wird. Für den Fall, daß sich im Luftabscheider während der Hämodialyse zusätzliche Luft ansammeln sollte, bleibt die Luftpumpe so lange in Betrieb, bis der obere Sensor Flüssigkeit detektiert.

Gemäß einer weiteren Ausführungsform besteht auch die Möglichkeit, anstatt mit Hilfe einer Luftpumpe mittels der Dialysierflüssigkeitspumpe, die zwischen dem Luftabscheider und der Bilanziereinrichtung angeordnet ist, die Luft in den Dialysierflüssigkeitsweg zu pumpen. Hierzu wird der Dialysierflüssigkeitsweg zwischen dem Ausgang des Dialysators und dem Luftabscheider mittels eines weiteren Ventils unterbrochen, um gegebenenfalls ein Ansaugen verbrauchter Dialysierflüssigkeit zu verhindern. Wenn in diesem Fall die Dialysierflüssigkeitspumpe weiterbetrieben wird, wird durch den Luftabscheider ebenfalls Luft in den Luftabscheider gesaugt. Zur Beschleunigung des Ansaugens kann die Pumpleistung der Dialysierflüssigkeitspumpe kurzzeitig erhöht werden. Zu diesem Zweck wird auch die Dialysierflüssigkeitspumpe von der Steuereinheit entsprechend geschaltet. Die Bestimmung der Luftmenge erfolgt dann ebenfalls über die beiden im Luftabscheider angeordneten Sensoren bzw. über den analogen Füllstandsmesser. Danach wird der Dialysierflüssigkeitsweg wieder geöffnet und die Ventile auf Dialysebetrieb geschaltet. Auch in diesem Fall wird anschließend während der Dialyse mittels der an den Luftabscheider angeschlossenen Luftpumpe die zuvor angesaugte Luftmenge wieder dem Dialysierflüssigkeitsweg entzogen. Dadurch steigt der Flüssigkeitsspiegel im Luftabscheider wieder an, und nachdem beide Sensoren Flüssigkeit detektieren ist die Ultrafiltration abgeschlossen, wobei eine dem Luftvolumen entsprechende Ultrafiltratmenge gewonnen wurde. Danach wird der Dialysierflüssigkeitsweg wieder mit Luft gefüllt und der Vorgang beginnt von neuem. Auf diese Weise ist es möglich, während der Dialyse kontinuierlich Ultrafiltrat zu entziehen.

Der Entzug der gewünschten Ultrafiltratmenge kann durch geeignete Steuerung der Luftpumpe auf beliebig viele Füllzyklen der Bilanzkammern verteilt werden. Durch geeignete Wahl des Kammervolumens des Luftabscheiders kann dieser das Mehrfache des Füllvolumens einer Bilanzkammer umfassen, so daß im Extremfall, wenn das Volumen des Luftabscheiders zwei bis drei Liter entspricht, dieser nur ein Mal mit Luft gefüllt werden muß.

Bei dem kontinuierlichen Entzug von Ultrafiltrat während der Dialyse ist die Flüssigkeitsmenge, die den Dialysator verläßt, um den Anteil des Ultrafiltrats vergrößert gegenüber der Menge, die an frischer Dialysierflüssigkeit dem Dialysator zugeführt wird. Da dieser zusätzliche Anteil durch den Entzug der Luft im Luftabscheider angesammelt wird, ändert sich gegenüber den bekannten Dialyseverfahren an der Arbeitsweise der Bilanzkammern nichts. Beim Ansaugen der Luft in den Luftabscheider wird die darin befindliche verbrauchte Dialysierflüssigkeit in den leeren Raum der Bilanzkammer gepumpt, die mit frischer Dialysierflüssigkeit gefüllt ist, wobei gleichzeitig diese frische Dialysierflüssigkeit in die andere Bilanzkammer gedrückt wird, wo wiederum die darin befindliche verbrauchte Dialysierflüssigkeit in den Auslauf verdrängt wird. Dies wird durch entsprechende Steuerung der Zuführ- und Abführventile erreicht.

Die erfindungsgemäße Vorrichtung weist keine Entnahmevorrichtung und somit keine Ultrafiltratpumpe auf. Der Ausgang des Dialysators ist in diesem Fall direkt mit dem Luftabscheider verbunden, der wiederum über die Dialysierflüssigkeitspumpe mit der Bilanziereinrichtung verbunden ist. Erfindungsgemäß wird die Steuereinrichtung zur Steuerung der Zuführ- und Abführventile der Bilanziereinrichtung sowie des Dialysatorventils und ggf. des zusätzlichen Absperrventils zwischen Dialysatorausgang und Luftabscheider so ausgebildet, daß das Ultrafiltrat in der vorher bestimmten Menge jeweils über eine Kammer der Bilanziereinrichtung zum Auslauf führbar ist. Hierbei werden die Absperrventile derart gesteuert, daß die abgeführte Ultrafiltratmenge jeweils einem Füllvolumen einer Bilanzkammer oder einem Mehrfachen entspricht.

Gemäß einer ersten Ausführungsform ist die Steuereinrichtung derart ausgebildet, daß sie zwischen zwei Hämodialysezyklen das Dialysatorventil schließt und die Absperrventile der Bilanziereinrichtung derart ansteuert, daß Ultrafiltrat in einen der beiden Räume einer der beiden Bilanzkammern mittels der Dialysierflüssigkeitspumpe gefördert werden kann, während die im anderen Raum dieser Bilanzkammer befindliche frische Dialysierflüssigkeit in die andere Bilanzkammer umgeleitet wird. Dies bedeutet, daß abwechselnd die Ventile zwischen Ultrafiltratmodus und Hämodialysemodus hin- und hergeschaltet werden. Unter Hämodialysemodus wird verstanden, daß die Ventile in den Zuführleitungen beispielsweise der beiden jeweils rechten Räume der Bilanzkammern und die Ventile der Abführleitungen der beiden jeweils linken Räume der Bilanzkammern geschlossen sind, während die Ventile in den Zuführ- und Abführleitungen der übrigen Räume geschlossen sind.

Beim Übergang zum Ultrafiltratmodus werden alle Ventile einer der beiden Bilanzkammern umgeschaltet, während bei der anderen Bilanzkammer lediglich die Ventile des linken Raumes umgeschaltet werden und die des rechten Raumes beibehalten werden.

Gemäß einer weiteren Ausführungsform ist die Steuereinheit zusätzlich zur Steuerung der Luftpumpe und/oder der Dialysierflüssigkeitspumpe ausgebildet. Die Schaltstellung der Ventile im Hämodialysemodus entspricht der in der oben beschriebenen Ausführungsform. Nach einem Hämodialysezyklus, bei dem auch gleichzeitig das Ultrafiltrat entzogen wird, werden die Ventile in den oben beschriebenen Ultrafiltratmodus geschaltet, der jetzt allerdings dazu dient, Luft in den Dialysierflüssigkeitsweg zu pumpen. Zu diesem Zweck steuert die Steuereinheit nicht nur die Absperrventile sondern auch die Luftpumpe und die Dialysierflüssigkeitspumpe sowie gegebenenfalls auch das weitere Absperrventil im Dialysierflüssigkeitsweg zwischen Dialysator und Luftabscheider an.

Um eine kontinuierliche Ultrafiltration durchzuführen, ist der Luftabscheider be- und entlüftbar ausgebildet. Gemäß einer besonderen Ausführungsform ist der Luftabscheider mit einer bidirektional arbeitenden Luftpumpe ausgestattet, die vor der Ultrafiltration die entsprechende Luftmenge in den Luftabscheider hineinpumpt und während der Ultrafiltration diese Luftmenge wieder aus dem Luftabscheider abpumpt. Damit das jeweilige Luftvolumen exakt bestimmt werden kann, sind am Luftabscheider zwei Sensoren derart im Abstand angeordnet, daß sie das Füllvolumen einer Bilanzkammer oder ein Vielfaches davon eingrenzen. Es ist auch möglich, anstelle dieser beiden Sensoren einen analog arbeitenden Füllstandsmesser einzubauen, dessen Abstand zwischen Maximal- und Minimalstellung ebenfalls einem Füllvolumen oder einem Mehrfachen des Füllvolumens einer Bilanzkammer entspricht.

Im Dialysierflüssigkeitsweg ist gemäß einer weiteren Ausführungsform außer dem Dialysatorventil zwischen dem Ausgang des Dialysators und dem Eingang des Luftabscheiders ein weiteres Ventil angeordnet.

Beispielhafte Ausführungsformen der Erfindung werden nachfolgend anhand der Zeichnung näher erläutert.

Es zeigen :
- Fig. 1: a,b, vereinfachte Prinzipschemata des Dialysierflüssigkeitswegs mit einer Bilanziereinrichtung gemäß einer ersten Ausführungsform;
- Fig.2: ebenfalls ein vereinfachtes Prinzipschema des Dialysierflüssigkeitswegs gemäß einer weiteren Ausführungsform;
- Fig.3: ebenfalls ein vereinfachtes Prinzipschema des Dialysierflüssigkeitswegs gemäß einer weiteren Ausführungsform.

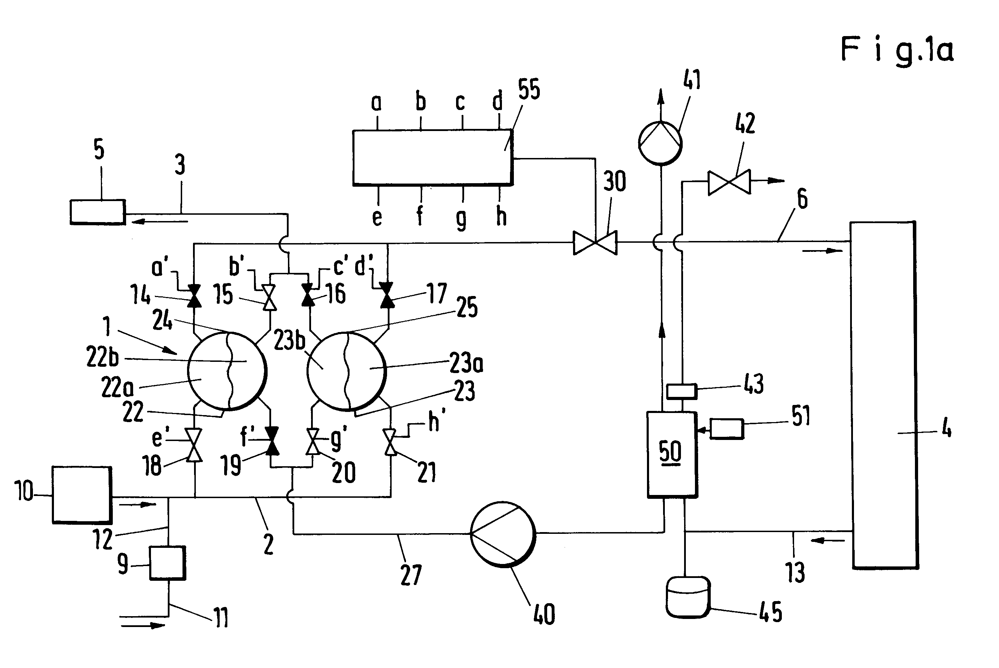

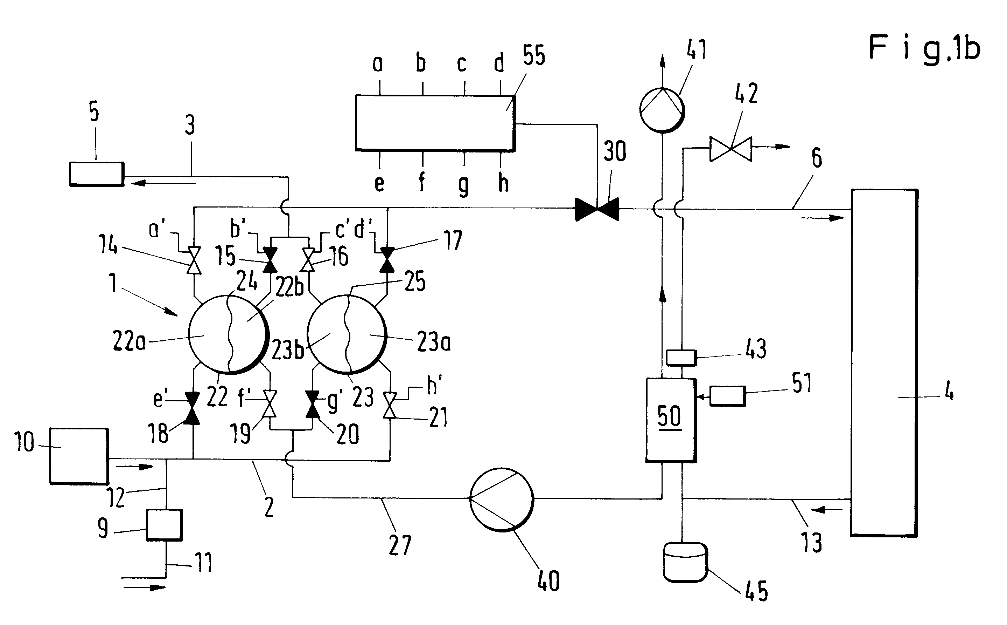

In der Fig. 1a ist der Dialysierflüssigkeitsweg schematisch dargestellt. Wichtigster Bestandteil dieser Anordnung ist die Bilanziervorrichtung 1. Sie umfaßt zwei miteinander gekoppelte Kammern 22 und 23, wobei jede Kammer 22 bzw. 23 durch ein bewegliches Element in Gestalt einer Membrane 24 bzw. 25 in zwei Räume 22a, 22b bzw. 23a und 23b unterteilt ist. Diese Bilanzkammern werden aus einer Dialysierflüssigkeitsquelle 10, die mit einer Pumpe versehen ist, sowie einer Dosiervorrichtung 9 mit frischer Dialysierflüssigkeit über die Leitung 2 versorgt. In dem hier gezeigten Beispiel ist der Raum 22a und der Raum 23a an diese Zuführleitung 2 angeschlossen, wobei zum Absperren jeweils ein Absperrventil 18 und ein Absperrventil 21 in dieser Zuführleitung 2 angeordnet ist. Diese Räume 22a, 23a der beiden Bilanzkammern 22 und 23 sind an ihrer Auslaßseite über weitere Absperrventile 14 bzw. 17 mit dem Dialysatorzuleitungszweig 6 verbunden, der zum Dialysator 4 führt. Zu diesem Zuleitungszweig 6 ist ein Dialysatorventil 30 eingeschaltet. Der Ausgang des Dialysators 4 ist über den Dialysatorableitungszweig 13, einen Luftabscheider 50 und eine Dialysierflüssigkeitsdruckmeßeinrichtung 45 sowie eine an den Luftabscheider 50 angeschlossene Dialysierflüssigkeitspumpe 40 wiederum mit der Bilanziereinrichtung 1 verbunden. Der Dialysierflüssigkeitsableitungszweig 27 zwischen der Dialysierflüssigkeitspumpe 40 und der Bilanziereinrichtung 1 teilt sich in zwei Leitungswege auf, die zu den Räumen 22b und 23b der Bilanzkammern 22 und 23 führen. In diesen beiden Leitungswegen sind vor den Bilanzkammern Absperrventile 19 und 20 angeordnet. Die Räume 22b und 23b sind über Absperrventile 15 und 16 mit der Ablaufleitung 3 verbunden, die zum Auslauf 5 führt.

Die Ventile 14-21 und 30 werden von einer Steuereinrichtung 55 angesteuert. Zu diesem Zweck weist die Steuereinrichtung Ausgänge a bis h auf, die mit den Ventilen 14 - 21 über die elektrischen Leitungen a′ bis h′ verbunden sind.

In der Fig. 1a sind die Ventile 14, 16, 17 und 19 dunkel (d.h. geöffnet) und die Ventile 15, 18, 20, 21 und das Dialysatorventil 30 hell (d.h. geschlossen) dargestellt. Diese Schaltsituation (sog. Ultrafiltratmodus) entspricht der ersten Ausführungsform des erfindungsgemäßen Verfahrens, bei der der Raum 22a vollständig mit frischer Dialysierflüssigkeit gefüllt ist und der Raum 23b vollständig mit verbrauchter Dialysierflüssigkeit. Wenn nun die Dialysierflüssigkeitspumpe 40 bei geschlossenem Dialysatorventil 30 in Betrieb ist, entsteht im Dialysator 4 ein Unterdruck, wodurch Ultrafiltrat dem Blutkreisweg (nicht dargestellt) entzogen wird. Dieses Ultrafiltrat wird über die Leitung 27 und das geöffnete Ventil 19 der Bilanzkammer 22 und dort dem Raum 22b zugeführt, wobei gleichzeitig frische Dialysierflüssigkeit aus dem Raum 22a über das geöffnete Ventil 14 und das geöffnete Ventil 17 dem Raum 23a der Bilanzkammer 23 zugeführt wird. Hierbei wird die in der Kammer 23b befindliche verbrauchte Dialysierflüssigkeit über das Ventil 16 dem Ablauf 5 zugeführt. Nachdem der Raum 22b der Bilanzkammer 22 vollständig mit Ultrafiltrat gefüllt ist, werden die Ventile 14 bis 21 und 30 von der Steuereinrichtung 55 derart angesteuert, daß die Ventile 14, 16, 19 und 21 geschlossen werden und die Ventile 15, 17, 18, 20 und 30 geöffnet werden (sog. Hämodialysemodus). Diese Schaltstellung ist in der Fig. 1b dargestellt. Bei dieser Ventilstellung wird dann die Hämodialyse durchgeführt, wie es aus der DE-PS 28 38 414 bekannt ist. Bei dem nachfolgenden Dialysezyklus wird dann der Raum 23b mit verbrauchter und der Raum 22a mit frischer Dialysierflüssigkeit gefüllt.

Für den Fall, daß sich in dem Dialysierflüssigkeitsweg im Luftabscheider 50 Luft abscheidet, fällt der Flüssigkeitsspiegel, so daß ein am Luftabscheider 50 angeordneter Luft/Flüssigkeitssensor 51 Luft detektiert. In diesem Fall wird die Pumpe 41 in Betrieb genommen, die dann die überflüssige Luft abpumpt, bis sie auf ein Signal des Sensors 51 hin desaktiviert wird.

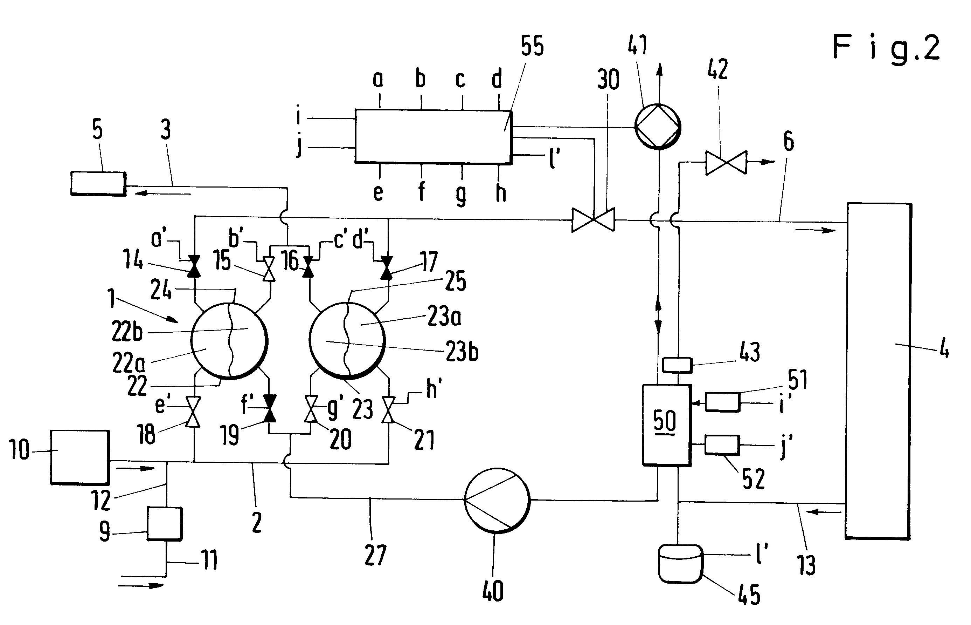

In der Fig. 2 ist eine weitere Ausführungsform dargestellt. In diesem Fall ist der Luftabscheider 50 mit zwei Sensoren, also oberem Sensor 51 und unterem Sensor 52 ausgestattet, die über die elektrischen Leitungen i′ und j′ mit den Eingängen i und j der Steuereinrichtung 55 verbunden sind. Anstelle eines analog arbeitenden Füllstandssensor bzw. anstelle zweier Sensoren 51, 52 kann auch ein einziger Sensor 51 in Kombination mit einer volumetrisch arbeitenden Pumpe eingesetzt werden. Da über den Dialysierflüssigkeitsdrucksensor 45 der Druck im System bekannt ist, kann die Pumpe 41 durch die Steuereinheit 55 so gesteuert werden, daß unter Berücksichtigung der allgemeinen Gasgleichung eine vorher bestimmbare Menge Dialysierflüssigkeit aus dem Luftabscheider 50 verdrängt wird. Zu diesem Zweck ist der Dialysierflüssigkeitsdrucksensor 45 über die elektrische Leitung 1′ mit dem Eingang 1 der Steuereinheit 55 verbunden.

Die Ventile 14, 16, 17, 19 bzw. 15, 18, 20, 21 sind dunkel bzw. hell dargestellt, was ebenfalls andeuten soll, daß diese Ventile von der Steuereinrichtung 55 auf offen bzw. geschlossen geschaltet worden sind. Die Schaltstellung der Ventile 14 bis 21 entspricht in diesem Fall der von Fig. 1a.

Vor Beginn der Ultrafiltration wird gemäß der hier gezeigten Ausführungsform zunächst über die als bidirektional arbeitende Luftpumpe 41, die ebenfalls an die Steuereinheit 55 angeschlossen ist, Luft in den Luftabscheider 50 gefördert, wobei die Pumprate so kontrolliert wird, daß der Druck am Dialysierflüssigkeitsdrucksensor 45 nicht ansteigt. Die Pumpe 41 fördert so lange Luft, bis Luft am unteren Sensor 52 detektiert wird. Die Sensoren 51 und 52 sind so positioniert, daß das zwischen ihnen liegende Volumen etwa dem Bilanzkammervolumen entspricht.

Die hierbei verdrängte Dialysierflüssigkeit wird von der Pumpe 40, deren Pumprate auf die der Luftpumpe 41 abgestimmt ist, über das Ventil 19 in den Raum 22b der Bilanzkammer 22 gepumpt. Bei vorgegebener Geschwindigkeit, entweder der Pumpe 40 oder der Pumpe 41, kann die jeweils andere Pumpe so gesteuert werden, daß der über den Dialysierflüssigkeitsdrucksensor 45 gemessene Dialysierflüssigkeitsdruck konstant bleibt.

Gleichzeitig wird die im Raum 22a der Bilanzkammer 22 befindliche frische Dialysierflüssigkeit über das Ventil 14 und das ebenfalls geöffnete Ventil 17 in den Raum 23a der zweiten Bilanzkammer 23 gepumpt.

Nachdem die gewünschte Luftmenge in den Luftabscheider 50 gepumpt worden ist, werden das Dialysatorventil 30 geöffnet und die Ventile 14 bis 21 von der Steuereinheit 55 in den Dialysemodus - wie vorstehend erwähnt und in Fig. 1b gezeigt ist - und die Luftpumpe 41 in den Entlüftungsbetrieb geschaltet. Dabei pumpt die Luftpumpe 41 über einen gewünschten Zeitraum die im Luftabscheider 50 befindliche Luft wieder aus dem Luftabscheider ab, bis der obere Sensor 51 wiederum Flüssigkeit detektiert und die Luftpumpe 41 desaktiviert. Auf diese Weise ist eine kontinuierliche Ultrafiltration während der Hämodialyse möglich.

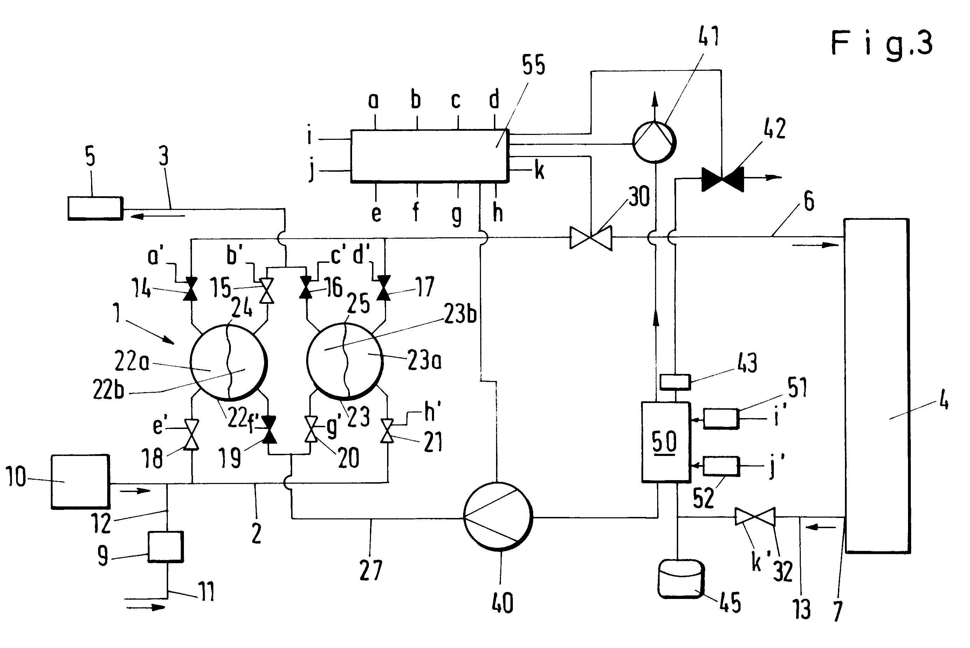

In der Fig. 3 ist eine weitere Ausführungsform dargestellt. In diesem Beispiel ist in dem Dialysatorableitungsweg 13, der den Ausgang 7 des Dialysators mit dem Luftabscheider 50 verbindet, ein zusätzliches Ventil 32 eingezeichnet, das über die Leitung k′ mit dem Eingang k der Steuereinheit 55 verbunden ist. Vor Beginn der Ultrafiltration wird, um ein Ansaugen von verbrauchter Dialysierflüssigkeit zu verhindern, dieses Ventil 32 von der Steuereinheit 55 geschlossen, während die Dialysierflüssigkeitspumpe 40 weiterläuft. Gleichzeitig ist die Luftpumpe 41 abgeschaltet und das Belüftungsventil 42 des Luftabscheiders 50, das ebenfalls an die Steuereinheit 55 angeschlossen ist, geöffnet. Dementsprechend ist Ventil 42 dunkel dargestellt. Dieses Ventil 42 bleibt so lange geöffnet, bis der untere Sensor 52 Luft detektiert. Die Schaltstellung der Ventile 14 bis 21 ist die gleiche wie die, die in der Fig. 2 dargestellt ist. Um die Luft möglichst schnell anzusaugen, kann die Pumprate der Pumpe 40 durch Ansteuerung der Steuereinheit 55 kurzzeitig erhöht werden.

Wenn sich die gewünschte Luftmenge im Luftabscheider 50 befindet, wird das Ventil 42 geschlossen und die Ventile 30 und 32 geöffnet. Gleichzeitig werden die Ventile 14 bis 21 in den Dialysemodus (s. Fig. 1b) geschaltet. Gleichzeitig wird in dieser Ausführungsform die einseitig wirkende Pumpe 41 von der Steuereinheit 55 in Betrieb genommen, die über einen vorher bestimmten Zeitraum die im Luftabscheider befindliche Luftmenge 50 wieder abpumpt. Dies geschieht so lange, bis der obere Sensor 51 wieder Flüssigkeit detektiert. Während dieses Zeitraumes wird durch den entstehenden Unterdruck Ultrafiltrat dem Blutweg entzogen. Diese Anordnung ist besonders präzise steuerbar, wenn anstelle der Sensoren 51 und 52 ein anlog arbeitender Füllstandssensor eingesetzt wird. Die Schaltung der Ventile während der Hämodialyse und der Ultrafiltration entspricht dann der bereits zu Figur 1a beschriebenen Schaltstellung.

## Patentansprüche

1. Verfahren zum Betreiben einer Hämodiafiltrationsvorrichtung, bei dem die Dialysierflüssigkeit durch eine an den Dialysator angeschlossene mit Absperrventilen versehene und Bilanzkammern aufweisende Bilanziereinrichtung geleitet wird und das Ultrafiltrat in einer genau vorherbestimmten Menge dem Dialysatorkreislauf entzogen wird, dadurch gekennzeichnet,
daß das Ultrafiltrat aus dem Dialysierflüssigkeitsweg über die Bilanziereinrichtung kontrolliert abgeführt wird, wobei zwischen zwei Hämodialysezyklen der Dialysierflüssigkeitsweg unterbrochen wird und die Absperrventile der Bilanziereinrichtung derart geschaltet werden, daß während des Einströmens zumindest des Ultrafiltrates in eine der Bilanzkammern die darin befindliche frische Dialysierflüssigkeit in die andere Bilanzkammer umgeleitet wird.

2. Verfahren nach Anspruch 1, dadurch gekennzeichnet, daß das Ultrafiltrat in solchen Mengen aus dem Dialysierflüssigkeitsweg abgeführt wird, die dem Füllvolumen der Bilanziereinrichtung oder einem Mehrfachen davon entsprechen.

3. Verfahren nach Anspruch 1 oder 2, dadurch gekennzeichnet, daß der Dialysierflüssigkeitsweg zwischen Bilanziereinrichtung und Dialysator für die Dauer des Ultrafiltratentzugs unterbrochen wird, wobei die gewünschte Ultrafiltratmenge in die Bilanziereinrichtung gefördert wird.

4. Verfahren nach Anspruch 1 oder 2, dadurch gekennzeichnet, daß das Ultrafiltrat kontinuierlich entzogen wird und zusammen mit der verbrauchten Dialysierflüssigkeit über die Bilanziereinrichtung abgeführt wird,
daß während der Unterbrechung des Dialysierflüssigkeitsweges zwischen der Bilanziereinrichtung und dem Dialysator dem Dialysierflüssigkeitsweg eine solche Menge Luft zugeführt wird, die dem Füllvolumen einer Bilanzkammer oder einem Mehrfachen davon entspricht, und
daß während der Hämodialyse die gleiche Luftmenge kontinuierlich dem Dialysierflüssigkeitsweg wieder entzogen wird, so daß eine Ultrafiltratmenge im Dialysierflüssigkeitsweg anfällt, die dieser Luftmenge entspricht.

5. Verfahren nach Anspruch 4, dadurch gekennzeichnet, daß die gewünschte Luftmenge über einen Luftabscheider zugeführt wird.

6. Verfahren nach Anspruch 4 oder 5, dadurch gekennzeichnet, daß die Luftmenge in einer solchen Rate zugeführt wird, daß der Druck im Dialysierflüssigkeitsweg nicht ansteigt.

7. Verfahren nach einem der Ansprüche 4 oder 5, dadurch gekennzeichnet, daß der Dialysierflüssigkeitsweg zusätzlich zwischen Dialysator und Luftabscheider unterbrochen wird und daß die gewünschte Luftmenge mittels der Dialysierflüssigkeitspumpe über den Luftabscheider angesaugt wird.

8. Vorrichtung zur Durchführung des Verfahrens gemäß Anspruch 1, mit einer Bilanziereinrichtung, die aus zwei durch ein verschiebbares Element getrennten Kammern besteht, die je eine Zuführleitung für frische und eine mit einem Auslauf verbundene Abführleitung für verbrauchte Dialysierflüssigkeit aufweisen, mit Absperrventilen, die in den Zuführ- und Abführleitungen angeordnet sind, mit wenigstens einer Einrichtung zur Endlagenerkennung des verschiebbaren Elementes, mit einer im Dialysierflüssigkeitsweg angeordneten Pumpe zum Fördern der verbrauchten Dialysierflüssigkeit, einem Dialysatorventil und einem Luftabscheider, mit einer Versorgungseinheit zur Bereitstellung von frischer Dialysierflüssigkeit, mit einem Dialysator und einer an die Absperrventile angeschlossenen Steuereinheit, dadurch gekennzeichnet,
daß die Steuereinheit (55) derart zur Steuerung und Schaltung der Absperrventile (14-21, 30) ausgebildet ist, daß das Ultrafiltrat in einer vorher bestimmten Menge über eine Kammer (24 oder 25) der Bilanziereinrichtung (1) zum Auslauf (5) führbar ist.

9. Vorrichtung nach Anspruch 8, dadurch gekennzeichnet, daß die Steuereinheit (55) die Absperrventile (14-21, 30) derart steuert, daß die abgeführte Ultrafiltratmenge jeweils dem Füllvolumen einer Bilanzkammer (22, 23) entspricht.

10. Vorrichtung nach Anspruch 8 oder 9, dadurch gekennzeichnet, daß die Steuereinheit (55) derart zur Steuerung und Schaltung der Absperrventile (14-21, 30) ausgebildet ist, daß während des Einströmens zumindest des Ultrafiltrates in eine der Bilanzkammern die darin befindliche frische Dialysierflüssigkeit in die andere Bilanzkammer (22, 23) umleitbar ist.

11. Vorrichtung nach einem der Ansprüche 8 bis 10, dadurch gekennzeichnet, daß der Luftabscheider (50) be- und entlüftbar ausgebildet ist.

12. Vorrichtung nach einem der Ansprüche 8 bis 11, dadurch gekennzeichnet, daß an dem Luftabscheider (50) eine bidirektional arbeitende Luftpumpe (41) angeschlossen ist.

13. Vorrichtung nach einem der Ansprüche 8 bis 12, dadurch gekennzeichnet, daß am Luftabscheider (50) ein unterer und ein oberer Sensor (51, 52) derart angeordnet sind, daß sie ein Volumen eingrenzen, das dem Füllvolumen einer Bilanzkammer (22, 23) oder einem Mehrfachen davon entspricht.

14. Vorrichtung nach einem der Ansprüche 8 bis 13, dadurch gekennzeichnet, daß am Luftabscheider (50) ein analog arbeitender Füllstandssensor angeordnet ist, dessen Minimal- und Maximalstellung ein Volumen eingrenzen, das dem Füllvolumen einer Bilanzkammer (22, 23) oder einem Mehrfachen davon entspricht.

15. Vorrichtung nach einem der Ansprüche 8 bis 14, dadurch gekennzeichnet, daß in dem Dialysatorableitungszweig (13) zwischen dem Ausgang (7) des Dialysators und dem Eingang des Luftabscheiders (50) ein Ventil (32) angeordnet ist.

## Claims

1. Method for operating a hemodiafiltration device, in which the dialyzer fluid is passed through a balancing installation, which is quipped with shut-off valves and balancing chambers and is connected to the dialyzer, and the ultrafiltrate is removed in an accurately predetermined amount from the dialyzer circuit, characterized in that the ultrafiltrate is removed from the dialysis fluid path through the balancing installation in a controlled manner, whereby between two hemodialysis cycles the dialysis fluid path is interrupted and the shut-off valves of the balancing installation are switched in such a way that during the inflow of at least the ultrafiltrate into one of the balancing chambers the fresh dialyzing fluid located therein is rerouted into the other balancing chamber.

2. Method according to Claim 1, characterized in that the ultrafiltrate is removed from the dialysis fluid path in such amounts which correspond to the filling volume of the balancing installation or is a multiple therof.

3. Method according to Claim 1 or 2, characterized in that the dialysis fluid path is interrupted between the balancing installation and the dialyzer for the duration of the removal of the ultrafiltrate, whereby the desired amount of ultrafiltrate is supplied to the balancing installation.

4. Method according to Claim 1 or 2, characterized in that the ultrafiltrate is removed continuously and is discharged together with the used dialysis fluid via the balancing installation, that during the interruption of the dialysis fluid path between the balancing installation and the dialyzer, air is supplied to the dialysis fluid path in such an amount which corresponds to the filling volume of one balancing chamber or is a multiple therof, and that during the hemodialysis, the same amount of air is removed continuously again from the dialysis fluid path, so that an amount of ultrafiltrate is obtained in the dialysis fluid path which corresponds to this amount of air.

5. Method according to Claim 4, characterized in that the desired amount of air is supplied via an air separator.

6. Method according to Claim 4 or 5, characterized in that the amount of air is supplied at such a rate that the pressure in the dialysis fluid path does not increase.

7. Method according to one of Claim 4 or 5, characterized in that the dialysis fluid path is additionally interrupted between the dialyzer and the air separator and that the desired amount of air is aspirated with the aid of the dialysis fluid pump via the air separator.

8. Device for carrying out the method according to Claim 1, with a balancing installation, which consists of two chambers that are separated by a sliding component, each chamber having one inlet pipe for fresh and one discharge pipe for removing the used dialysis fluid, connected to an outlet, with shut-off valves, arranged in the inlet and discharge pipes, with at least one installation for recognizing the final position of the sliding component, with a pump arranged in the dialysis fluid path for transporting the used dialysis fluid, a dialyzer valve and an air separator, with a supply unit for providing fresh dialysis fluids, with a dialyzer and a control unit connected to the shut-off valves, characterized in that the control unit (55) is designed for the control and switching of the shut-off valves (14-21, 30) in such a way that the ultrafiltrate can be discharged in a previously determined amount through a chamber (24 or 25) of the balancing installation (1) to the outlet (5).

9. Device according to Claim 8, characterized in that the control unit (55) controls the shut-off valves (14-21, 30) in such a way that the amount of discharged ultrafiltrate always corresponds to the filling volume of one balancing chamber (22, 23).

10. Device according to Claim 8 or 9, characterized in that the control unit (55) is designed for the control and switching of the shut-off valves (14-21, 30) in such a way that during the inflow of at least the ultrafiltrate into one of the balancing chambers, the fresh dialysis fluid located therein can be rerouted to the other balancing chamber (22, 23).

11. Device according to one of Claims 8 or 10, characterized in that the air separator (50) is designed so that the air can be introduced into it or removed from it.

12. Device according to one of Claims 8 to 11, characterized in that a bidirectionally operating air pump (41) is connected to the air separator (50).

13. Device according to one of Claims 8 to 12, characterized in that a lower and an upper sensor (51, 52) are arranged at the air separator (50) in such a way they limit a volume that corresponds to the filling volume of one balancing chamber (22, 23) or to a multiple of this.

14. Device according to one of Claims 8 to 13, characterized in that an analog level sensor is arranged at the air separator (50), the minimum and maximum positions of which define a volume that corresponds to the filling volume of one balancing chamber (22, 23) or to a multiple thereof.

15. Device according to one of Claims 8 to 14, characterized in that a valve (32) is arranged in the dialyses discharge branch (13) between the outlet (7) of the dialyzer and the inlet of the air separator (50).

## Revendications

1. Procédé pour faire fonctionner un dispositif d'hémodiafiltration, selon lequel on amène le liquide de dialyse à traverser un dispositif d'établissement de bilan, qui est raccordé au dialyseur et comporte des soupapes de sectionnement et des chambres d'établissement du bilan, et selon lequel l'ultrafiltrat est prélevé en une quantité pouvant être prédéterminée de façon précise, à partir du circuit du dialyseur, caractérisé en ce
que l'ultrafiltrat est évacué de la voie de circulation du liquide de dialyse, d'une manière contrôlée par l'intermédiaire du dispositif d'établissement du bilan, auquel cas le trajet de circulation du liquide de dialyse est interrompu entre deux cycles d'hémodialyse et les soupapes de sectionnement du dispositif d'établissement du bilan sont commutées de telle sorte que pendant l'introduction au moins de l'ultrafiltrat dans l'une des chambres d'établissement du bilan, le liquide de dialyse frais situé dans cette chambre est renvoyé dans l'autre chambre d'établissement du bilan.

2. Procédé selon la revendication 1, caractérisé en ce que l'ultrafiltrat est prélevé de la voie de circulation du liquide dialyse, en des quantités qui correspondent au volume de remplissage du dispositif d'établissement du bilan ou à un multiple de cette valeur.

3. Procédé selon la revendication 1 ou 2, caractérisé en ce que la voie de circulation du liquide de dialyse est interrompue entre le dispositif d'établissement du bilan et le dialyseur pendant la durée du prélèvement de l'ultrafiltrat, la quantité désirée d'ultrafiltrat étant introduite dans le dispositif d'établissement du bilan.

4. Procédé selon la revendication 1 ou 2, caractérisé en ce que l'ultrafiltrat est prélevé continûment et est évacué conjointement avec le liquide de dialyse utilisé, par l'intermédiaire du dispositif d'établissement du bilan,
que pendant l'interruption du trajet de circulation du liquide de dialyse entre le dispositif d'établissement du bilan et le dialyseur, une quantité d'air, qui correspond au volume de remplissage d'une chambre d'établissement du bilan ou un multiple de ce volume, est envoyée au dialyseur,
que pendant l'hémodialyse, la même quantité d'air est à nouveau prélevée continûment de la voie de circulation du liquide de dialyse, de sorte qu'une quantité d'ultrafiltrat, qui correspond à cette quantité d'air, pénètre dans la voie de circulation du liquide de dialyse.

5. Procédé selon la revendication 4, caractérisé en ce que la quantité d'air désirée est envoyée par l'intermédiaire d'un séparateur d'air.

6. Procédé selon la revendication 4 ou 5, caractérisé en ce que l'air est envoyé avec un débit tel que la pression dans la voie de circulation du liquide de dialyse n'augmente pas.

7. Procédé selon l'une des revendications 4 ou 5, caractérisé en ce que la voie de circulation du liquide de dialyse est interrompue de façon supplémentaire entre le dialyseur et le séparateur d'air et que la quantité d'air désirée est aspirée au moyen de la pompe d'entraînement du liquide de dialyse par l'intermédiaire du séparateur d'air.

8. Dispositif pour la mise en oeuvre du procédé selon la revendication 1, comportant un dispositif d'établissement du bilan, qui est constitué de deux chambres séparées par un élément mobile et qui possèdent chacune une canalisation d'arrivée pour le liquide frais de dialyse et une canalisation d'évacuation, raccordée à une sortie, pour le liquide de dialyse consommé, et comportant des soupapes de sectionnement, qui sont disposées dans les canalisations d'évacuation, au moins un dispositif pour identifier la position finale de l'élément mobile, une pompe disposée dans la voie de circulation du liquide de dialyse pour entraîner le liquide de dialyse consommé, une soupape du dialyseur et un séparateur d'air, une unité d'alimentation pour préparer du liquide de dialyse frais, un dialyseur et une unité de commande raccordée aux soupapes de sectionnement,
caractérisé par le fait
que l'unité de commande (55) est conçue pour la commande et la commutation des soupapes de sectionnement (14-21,30) de telle sorte que l'ultrafiltrat peut être envoyé en une quantité déterminée de façon préalable, à la sortie (5), par l'intermédiaire d'une chambre (24 ou 25) du dispositif d'établissement du bilan (1).

9. Dispositif selon la revendication 8, caractérisé en ce que l'unité de commande (55) commande les soupapes de sectionnement (14-21,30) de telle sorte que la quantité d'ultrafiltrat évacuée correspond respectivement au volume de remplissage d'une chambre d'établissement du bilan (22,23).

10. Dispositif selon la revendication 8 ou 9, caractérisé en ce que l'unité de commande (55) est conçue pour la commande et la commutation des soupapes de sectionnement (14-21,30) de telle sorte que pendant l'introduction au moins de l'ultrafiltrat dans l'une des chambres d'établissement du bilan, le liquide de dialyse frais situé dans cette chambre, peut être renvoyé dans l'autre chambre d'établissement du bilan (22,23).

11. Dispositif selon l'une des revendications 8 à 10, caractérisé en ce que le séparateur d'air (50) est agencé de manière à pouvoir être aéré et désaéré.

12. Dispositif selon l'une des revendications 8 à 11, caractérisé en ce qu'une pompe à air à fonctionnement bidirectionnel (41) est raccordée au séparateur d'air (50).

13. Dispositif selon l'une des revendications 8 à 12, caractérisé en ce que dans le séparateur d'air (50) sont disposés un capteur inférieur et un capteur supérieur (51,52), de telle sorte que ces capteurs limitent un volume qui correspond au volume de remplissage d'une chambre de formation d'établissement du bilan (22,23) ou un multiple de ce volume.

14. Dispositif selon l'une des revendications 8 à 13, caractérisé en ce que dans le séparateur d'air (50) est disposé un capteur de l'état de remplissage, qui travaille de façon analogique et dont le réglage minimum et le réglage maximum limitent un volume, qui correspond au volume de remplissage d'une chambre d'établissement du bilan (22,23) ou à un multiple de ce volume.

15. Dispositif selon l'une des revendications 8 à 14, caractérisé en ce que dans la branche de dérivation (13) du dialyseur, une soupape (32) est disposée entre la sortie (7) du dialyseur et l'entrée du séparateur d'air (50).
